# EUROPÄISCHE PATENTANMELDUNG

(11) **EP 3 993 317 A1**
(43) Veröffentlichungstag der Anmeldung: **04.05.2022**
(21) Anmeldenummer: 21204615.5
(22) Anmeldetag: 26.10.2021
(51) Int. Cl.: H04L 9/40, G05B 19/042, G05B 19/418

(54) **MESSSYSTEM, KOMMUNIKATIONSKOMPONENTE, VORRICHTUNG, VERFAHREN UND COMPUTERPROGRAMM FÜR EINE KOMMUNIKATIONSKOMPONENTE EINES MESSSYSTEMS ZUM SYNCHRONISIEREN VON ZUGANGSDATEN**

(30) Priorität: 02.11.2020 DE 102020128744
(71) Anmelder: Dräger Safety AG & Co. KGaA, 23560 Lübeck (DE)
(72) Erfinder: Wahlbrink, Günter, 23558 Lübeck (DE); Furuta, Jun, 23558 Lübeck (DE); Sturm, Hannes, 23558 Lübeck (DE)
(74) Vertreter: Guthöhrlein, Gerhard

(57) **Zusammenfassung**

Ausführungsbeispiele beziehen sich auf ein Messsystem (500), eine Kommunikationskomponente (200; 300a; 300b; 300c; 600; 700), eine Vorrichtung (20), ein Verfahren (10) und ein Computerprogramm für eine Kommunikationskomponente (200; 300a; 300b; 300c; 600; 700) eines Messsystems (500). Das Messsystem (500) weist ein oder mehrere weitere Kommunikationskomponenten (200; 300a; 300b; 300c; 600; 700) auf. Das Verfahren (10) für die Kommunikationskomponente (200; 300a; 300b; 300c; 600; 700) des Messsystems (500) umfasst. Verwalten (11) zumindest eines personalisierten Nutzers mit Zugriffsrechten in dem Messsystem (500) und Speichern (12) von Zugangsdaten für den zumindest einen personalisierten Nutzer. Das Verfahren (10) umfasst ferner Synchronisieren (13) der Zugangsdaten des zumindest einen personalisierten Nutzers mit den ein oder mehreren weiteren Kommunikationskomponenten (200; 300a; 300b; 300c; 600; 700).

## Beschreibung

Die vorliegende Erfindung bezieht sich auf ein Messsystem, eine Kommunikationskomponente, eine Vorrichtung, ein Verfahren und ein Computerprogramm für eine Kommunikationskomponente eines Messsystems, insbesondere, aber nicht ausschließlich, auf ein Konzept zur Verwaltung von Zugangsdaten in einem Gasmesssystem.

Messsysteme sind zumeist aus verschiedenen Komponenten aufgebaut, die miteinander über verschiedene Kommunikationswege und mehrere Hierarchieebenen hinweg kommunizieren. Insbesondere im industriellen Umfeld werden Messsysteme für vielfältige Zwecke eingesetzt. Zur Kontrolle oder Steuerung und Regelung verschiedener Prozessparameter, beispielsweise Temperatur, Druck, Durchfluss oder Gaskonzentration, werden in der Regel Sensoren direkt am Ort der zu messenden Größe eingesetzt. Transmitter bereiten diese Messsignale in geeigneter Form auf und senden diese häufig an zentrale Auswertesysteme, wie beispielsweise eine SPS (Speicherprogrammierbare Steuerung), einen Regler oder einen Kontroller, der an zentraler Stelle, beispielsweise einer Leitwarte, eine Überwachung auch mehrerer Messtellen, oder einen direkten Eingriff in den Prozess durch eine Regelung ermöglicht. Eine zunehmende Kommunikation dieser verschiedenen Komponenten eines Messsystems untereinander oder auch in externe Systeme wie übergeordnete Speicher und Internetbereiche ermöglichen umfassende Möglichkeiten zur Kontrolle, Archivierung, Konfiguration und Speicherung umfassender Systemdaten einer Messeinrichtung auch von dezentralen Zugriffspunkten.

Beispielsweise kann eine Architektur eines Messsystems vorsehen, dass ein oder mehrere Sensoren jeweils einem Kontroller zugeordnet sind. Im Bereich der stationären Gaswarntechnik wird in der Regel von einem Transmitter gesprochen. Ein Sensor zur Messung einer Gaskonzentrationen liefert Messwerte an einen Transmitter, der nach geeigneter Aufbereitung und Digitalisierung der Messwerte, diese zusammen mit Statusinformationen über eine Prozessschnittstelle an geeignete Empfänger sendet.

Ein Sensor ist beispielsweise ein Messaufnehmer, der Teil einer Messkette sein kann, und zur Umwandlung von physikalischen und/oder chemischen Messgrößen (z.B. Gaskonzentrationen) in ein elektrisches Signal ausgebildet ist. Er kann weitere Elemente wie Signalverstärker, Linearisierung, Normierung, auch in Form komplexer digitaler Verarbeitung, enthalten. Ein Transmitter kann zum Betrieb und zur Versorgung eines Sensors oder Messaufnehmers ausgebildet sein. Er kann auch beispielsweise Teile der Signalverarbeitung der vom Sensor kommenden Signale enthalten, sofern diese nicht im Sensor bereits enthalten sind. Ein Transmitter kann eine Schnittstelle zur Wartung und Konfiguration umfassen, wie beispielsweise eine Anzeige oder ein Display. Außerdem kann ein Transmitter zur Bereitstellung und Übertragung eines in der Regel normierten Messsignals (z.B. 4-20 mA, 0-10 V, digital) plus gegebenenfalls spezielle Zustände des Systems wie Fehler, Warnungen, Messbereichsüberschreitungen etc. ausgebildet sein.

Insofern kann im Folgenden auch Bezug auf den Transmitter genommen werden. Ein Kontroller oder SPS ist dabei eine Implementierung von einer Auswertungs- und/oder Steuerungseinheit. Der Begriff Kontroller wird daher stellvertretend für eine Implementierung einer Auswertungs- und/oder Steuerungseinheit benutzt. Die mehreren Kontroller können dann direkt miteinander kommunizieren und/oder sie kommunizieren mit einer weiteren Verwaltungseinheit, die wiederum den Kontrollern übergeordnet sein kann.

Beispielsweise können die einzelnen Komponenten einer Gaswarnanlage, die mehrheitlich örtlich voneinander getrennt implementiert sein können, gegen unbefugten Zugriff geschützt sein. Bei einer komplexen Messkette ausgehend von einem Sensor bis hin zu messwertverarbeitenden und darstellenden übergeordneten Auswerte- und Speichersystemen gibt es eine Vielzahl von Zugriffspunkten, die alle gegen unberechtigtes Zugreifen oder Manipulation geschützt werden sollen, aber die gleiche Messkette betreffen.

Üblicherweise geschieht das mit der Eingabe eines Pins oder Passworts, um beispielsweise sicherheitsrelevante Einstellungen oder Kalibrierungen vorzunehmen. Um einen spezifischen Zugang zu ermöglichen, sind häufig verschiedene Zugriffsrechte vorgesehen. So gibt es beispielsweise unterschiedliche (rollenbasierte) Berechtigungsebenen für Bediener, Wartungspersonal oder Administratoren, auf die unterschiedliche Einflussebenen (Zugriffsberechtigungen), beispielsweise bei einem Transmitter oder Sensor, abgebildet werden. Bei einem Auswertesystem, wie beispielsweise einem Computer, gibt es bereits verschiedene physikalische Zugriffsverfahren, die über Eingabe von Passwörtern realisiert sind oder auch über Verfahren die Fingerabdrücke oder visuelle Profile eines Benutzers erkennen.

In konventionellen Systemen werden beispielsweise unterschiedliche Nutzerverwaltungen bei den Komponenten verwendet, z.B. bei Kontrollern und Transmittern/Sensoren. Diese sind rollenbasiert aufgebaut und unterscheiden sich zumeist hinsichtlich der Zugriffsrechte, zumindest derer von Administratoren im Vergleich zu anderen Nutzern.

Bei Applikationen mit Internetanbindung kann teilweise ein einheitlicher Username(Benutzername)/Passwort für Cloudanwendungen sowie aller zugehörigen Endgeräte mit "Internetfähigkeit" verwendet werden. Im industriellen Umfeld steht jedoch auf der Feldebene aus verschiedenen Gründen (Explosionsschutz, Energieverbrauch) nicht jedem Gerät oder ggf. keinem Gerät eine globale Nutzerverwaltung zur Verfügung. Eine direkte Ursache hierfür ist, dass die Geräte auf Feldebene nicht über eine Konnektivitätsfunktion verfügen, was wiederum im begrenzten Energiebudget - bedingt durch geringe Kapazität der Prozessschnittstelle oder den Explosionsschutz - begründet liegt. Neuartige Elektronikkomponenten (z.B. Kontroller) können jedoch ermöglichen, bei gleichem Energiebudget diese Funktionen zu realisieren. Es werden daher verschiedene Benutzerverwaltungen und ggf. Zugriffsrechteverwaltungen im gleichen System betrieben. Dies kann je nach Ausdehnung und Implementierung eines Messsystems einen erheblichen Aufwand bedeuten, wenn beispielsweise Zugangsdaten oder Zugriffsrechte systemweit geändert werden. Sicherheitsaspekte sind dabei ebenfalls wesentlich.

Vor diesem Hintergrund besteht daher ein Bedarf ein verbessertes Konzept zur Nutzerverwaltung in Messsystemen zu schaffen. Diesem Bedarf kommen die Gegenstände der anhängigen unabhängigen Ansprüche nach.

Ausführungsbeispiele basieren auf dem Kerngedanken, beispielsweise in einem System mit eingangs beschriebener Architektur, zwischen Kontroller und Transmitter/Sensor eine einheitliche, personalisierte und synchronisierte Nutzerverwaltung einzuführen. Es ist eine Erkenntnis, dass eine Synchronisation zwischen Kontroller und Transmitter/Sensor oder zwischen Transmitter/Sensor und Transmitter/Sensor erfolgen kann, sodass die Zugangsdaten zwischen den sich synchronisierenden Komponenten vereinheitlicht werden. Dies kann insbesondere bei Kommunikation über digitale Feldbusse (z.B. 4-20mA und HART (von engl. Highway Addressable Remote Transducer - ein standardisiertes Kommunikationssystem zum Aufbau industrieller Feldbusse)) die Nutzerverwaltung erleichtern.

Ausführungsbeispiele schaffen ein Verfahren für eine Kommunikationskomponente eines Messsystems. Das Messsystem weist ein oder mehrere weitere Kommunikationskomponenten auf. Das Verfahren umfasst ein Verwalten zumindest eines personalisierten Nutzers mit Zugriffsrechten in dem Messsystem und ein Speichern von Zugangsdaten für den zumindest einen personalisierten Nutzer. Das Verfahren umfasst ferner ein Synchronisieren der Zugangsdaten des zumindest einen personalisierten Nutzers mit den ein oder mehreren weiteren Kommunikationskomponenten. Die Kommunikationskomponente umfasst dabei einen Kontroller und die ein oder mehreren weiteren Kommunikationskomponenten umfassen ein oder mehrere Transmitter. Das Verfahren umfasst darüber hinaus ein Erhalten aktualisierter Zugangsdaten an den ein oder mehreren Transmittern oder an einer anderen mit dem Kontroller kommunizierenden Kommunikationskomponente und ein Vereinheitlichen der aktualisierten Zugangsdaten unter den ein oder mehreren Kommunikationskomponenten. Ausführungsbeispiele können eine automatisierte Vereinheitlichung auch bei Aktualisierungen der Zugangsdaten vorsehen.

Das Verwalten eines personalisierten Nutzerprofils erlaubt eine bessere Anpassung eines Zugriffsrechteprofils auf einen individuellen Nutzer als dies bei einer rollenbasierten Nutzerverwaltung möglich wäre. Ausführungsbeispiele können so eine Vereinheitlichung der Zugangsdaten unter den ein oder mehreren Kommunikationskomponenten erreichen und damit administrativen Aufwand, der bei getrennten Nutzerverwaltungen notwendig wäre, einsparen. Wenn beispielsweise ein Nutzer Nutzerdaten ändert (z.B. Passwort), können diese entsprechende Änderungen auf allen anderen Transmittern, Kontrollern und ggf. Applikationen in der Cloud oder in einer lokalen IT (Informationstechnik) automatisiert auslösen und müssen nicht zeitaufwendig an allen Einzelgeräten vorgenommen werden. Ein Nutzer mit globalen Zugriffsrechten, beispielsweise ein Administrator, braucht demnach nicht mehr viele unterschiedliche Passwörter zu verwalten, da ein identischer, für das Messsystem gültiger Zugriffscode entsprechend seiner Zugriffsberechtigungsebene nunmehr ausreichend ist.

Eine Nutzerverwaltung kann in Ausführungsbeispielen personalisiert erfolgen. Insgesamt kann die Sicherheit in Ausführungsbeispielen erhöht werden, da Nutzerdaten nicht mehr aufwendig global gepflegt werden müssen und schnelle Änderungen bei Bedarf durchgeführt werden können. Durch eine entsprechende Dokumentation kann auf Nutzerebene nachvollziehbar gemacht werden, wer welche Änderungen (z.B. Konfiguration) zu welchem Zeitpunkt durchgeführt hat.

Das Messsystem kann beispielsweise eine Gasmessanlage umfassen, sodass eine entsprechende personalisierte Nutzerverwaltung in der Gasmessanlage implementiert ist. Das Messsystem kann eine Gaswarnanlage umfassen, sodass entsprechende Warnkomponenten im Rahmen des oben beschriebenen Verfahrens mit synchronisiert werden können. Generell ist hier und im Folgenden eine Trennung von Gasmesssystem und Gaswarnsystem weniger technisch zu sehen als mehr sprachgebräuchlich. Bei einer Gaswarnanlage kann beispielsweise die Auslösung verschiedener Ereignisse oder Schwellen eher von Interesse sein und weniger die Kenntnis eines Messignals.

In einigen weiteren Ausführungsbeispielen kann das Synchronisieren ferner ein Synchronisieren der Zugriffsrechte des zumindest einen personalisierten Nutzers umfassen. Ausführungsbeispiele können so auch eine Vereinheitlichung der Zugriffsrechte erlauben.

Die Zugriffrechte können auf einer dem zumindest einen personalisierten Nutzer zugewiesenen Rolle basieren. Beispielsweise können rollenbasierte Basisrechteprofile die Erstellung eines Zugriffsrechteprofils für einen (neuen) Nutzer erleichtern.

Das Messsystem kann in Ausführungsbeispielen zumindest einen Kontroller und ein oder mehrere mit dem Kontroller digital kommunizierende Transmitter umfassen, wobei die Transmitter mit Gasmesssensoren gekoppelt sind. Ausführungsbeispiele können eine Synchronisation von personalisierten Zugangsdaten zwischen Kontrollern und Transmittern erlauben.

Das Synchronisieren kann zumindest teilweise über einen digitalen Feldbus erfolgen. Ausführungsbeispiele können eine Kommunikation von Zugangsdaten oder deren Änderungen auch über digitale Feldbusse erlauben.

In manchen Ausführungsbeispielen kann ein Dokumentieren von Änderungen der Zugangsdaten erfolgen, sodass eine Änderungshistorie nachvollziehbar gemacht werden kann.

Die Kommunikationskomponente kann beispielsweise einen Kontroller und die ein oder mehreren weiteren Kommunikationskomponenten ein oder mehrere Transmitter umfassen. Das Synchronisieren der Zugangsdaten kann dann ein Vereinheitlichen der Zugangsdaten an den mehreren Transmittern durch den Kontroller umfassen. In manchen Ausführungsbeispielen kann die Koordination der Synchronisation durch den Kontroller erfolgen.

Das Erhalten der aktualisierten Zugangsdaten kann an dem Kontroller, an einem Transmitter oder an einer anderen mit dem Kontroller kommunizierenden Kommunikationskomponente erfolgen. Damit können Ausführungsbeispiele erlauben, von mehreren Zugangspunkten des Messsystems oder sogar von allen Zugangspunkten des Messsystems aus aktualisierte Zugangsdaten zu erhalten. Die andere mit dem Kontroller kommunizierende Kommunikationskomponente kann beispielsweise eine Leitwarte in einem zu dem Messsystem lokalen IT-System oder einen Zugangsknoten in einer Cloud umfassen.

Zumindest einige Ausführungsbeispiele können ein Vereinheitlichen der aktualisierten Zugangsdaten an allen für einen Nutzerzugang geeigneten Kommunikationskomponenten umfassen. Gegebenenfalls kann so eine automatische systemweite Synchronisation/Aktualisierung der Zugangsdaten erreicht werden.

Das Verfahren kann darüber hinaus ein Verwalten von personalisierten Zugriffsrechten für den zumindest einen personalisierten Nutzer umfassen. Manche Ausführungsbeispiele können ein Verfahren zur synchronisierten Verwaltung von personalisierten Zugangsdaten und Zugriffsrechten bereitstellen.

Ein weiteres Ausführungsbeispiel ist ein Computerprogramm mit einem Programmcode zur Durchführung eines der hierin beschriebenen Verfahren, wenn der Programmcode auf einem Computer, einem Prozessor oder einer programmierbaren Hardwarekomponente ausgeführt wird. Ein maschinenlesbarer Datenträger mit einem solchen Programmcode ist ein weiteres Ausführungsbeispiel.

Ausführungsbeispiele schaffen darüber hinaus eine Vorrichtung für eine Kommunikationskomponente eines Messsystems. Die Vorrichtung umfasst eine Schnittstelle, die zur Kommunikation in einem Netzwerk ausgebildet ist und ein Kontrollmodul, das zur Durchführung eines der hierin beschriebenen Verfahren ausgebildet ist.

Ein weiteres Ausführungsbeispiel ist eine Kommunikationskomponente für ein Messsystem mit einer solchen Vorrichtung. Die Kommunikationskomponente kann einen oder mehrere Transmitter und/oder einen oder mehrere Kontroller umfassen.

Darüber hinaus schaffen Ausführungsbeispiele ein Messsystem mit mehreren Kommunikationskomponenten wie hierin beschrieben, wobei zumindest eine erste Kommunikationskomponente einen Kontroller umfasst und zumindest eine zweite Kommunikationskomponente einen Transmitter umfasst. Gemäß obiger Erläuterung kann das Messsystem eine Gasmessanlage und/oder eine Gaswarnanlage umfassen.

Schließlich schaffen Ausführungsbeispiele auch ein System mit einer Mehrzahl von Kommunikationskomponenten gemäß dieser Beschreibung.

Einige Beispiele von Vorrichtungen und/oder Verfahren werden nachfolgend bezugnehmend auf die beiliegenden Figuren näher erläutert.
Fig. 1 zeigt ein Blockschaltbild eines Ausführungsbeispiels eines Verfahrens für eine Kommunikationskomponente eines Messsystems;
Fig. 2 zeigt ein Blockschaltbild eines Ausführungsbeispiels einer Vorrichtung für eine Kommunikationskomponente eines Messsystems, eines Ausführungsbeispiels einer Kommunikationskomponente und ein Ausführungsbeispiel eines Messsystems;
Fig. 3 zeigt ein Blockschaltbild eines Ausführungsbeispiels eines Messsystems; und
Fig. 4 zeigt mögliche Kommunikationsszenarien in einem Ausführungsbeispiel.

Verschiedene Beispiele werden nun ausführlicher Bezug nehmend auf die beiliegenden Figuren beschrieben. In den Figuren können die Stärken von Linien, Schichten und/oder Bereichen zur Verdeutlichung übertrieben sein.

Weitere Beispiele können Modifikationen, Entsprechungen und Alternativen abdecken, die in den Rahmen der Offenbarung fallen. Gleiche oder ähnliche Bezugszeichen beziehen sich in der gesamten Beschreibung der Figuren auf gleiche oder ähnliche Elemente, die bei einem Vergleich miteinander identisch oder in modifizierter Form implementiert sein können, während sie die gleiche oder eine ähnliche Funktion bereitstellen.

Es versteht sich, dass, wenn ein Element als mit einem anderen Element "verbunden" oder "gekoppelt" bezeichnet wird, die Elemente direkt, oder über ein oder mehrere Zwischenelemente, verbunden oder gekoppelt sein können. Wenn zwei Elemente A und B unter Verwendung eines "oder" kombiniert werden, ist dies so zu verstehen, dass alle möglichen Kombinationen offenbart sind, d. h. nur A, nur B sowie A und B, sofern nicht explizit oder implizit anders definiert. Eine alternative Formulierung für die gleichen Kombinationen ist "zumindest eines von A und B" oder "A und/oder B". Das Gleiche gilt, mutatis mutandis, für Kombinationen von mehr als zwei Elementen.

Mit zunehmender Sensibilität zur IT Sicherheit bedürfen Messsysteme allgemein mehr und mehr einer Authentifizierung des Benutzers, sollte dieser auf die zur Verfügung gestellten Daten lesend (z.B. Auslesen von Messwerten oder Konfigurationsdaten) oder schreibend (z.B. Konfiguration ändern, Kalibrieren v. Sensoren) zugreifen wollen. Die Authentifizierung kann z.B. durch die Kombination von Nutzername und einem Kennwort erfolgen. Aufgrund der Vielzahl von verschiedenen Auswerteebenen ergibt sich daraus in konventionellen Systemen eine Vielzahl von unabhängigen und unterschiedlichen Zugriffskonzepten und Passwörtern für eine technisch zusammenhängende Messkette. Ausführungsbeispiele ermöglichen eine einfachere Verwaltung der Zugriffssituation über diese ganze Messkette.

In konventionellen Systemen kann die Nutzerverwaltung uneinheitlich sein, d.h. ein Nutzer kann verschiedene Zugangsdaten an verschiedenen Komponenten desselben Messsystems haben, das kann erhöhte Verwaltungsaufwände zur Folge haben. Darüber hinaus kann die Nutzerverwaltung unsynchronisiert sein, d.h. sollte ein Nutzer Nutzerdaten ändern, beispielsweise ein Passwort, wird dieses keine Änderungen auf alle anderen Transmitter, Controller und ggf. Applikationen in Cloud oder lokaler IT haben. Änderungen der Nutzerdaten werden dann ggf. zeitaufwendig an allen Einzelgeräten vorgenommen werden. Ein Nutzer mit globalen Zugriffsrechten beispielsweise ein Administrator, muss dann viele konventionelle unterschiedliche Passwörter verwalten obwohl ein identischer, für das Messsystem gültiger Zugriffscode entsprechend seiner Zugriffsberechtigungsebene ausreichend wäre.

Darüber hinaus ist eine Nutzerverwaltung nicht personalisiert und i.d.R. gibt es lediglich eine rollenbasierte Nutzerverwaltung. Ein personalisierter Rechtentzug ist damit ausgeschlossen. Diese Punkte können insbesondere Auswirkungen auf die Sicherheit haben, da Nutzerdaten nur aufwendig global gepflegt werden können (z.B. schnelle Änderungen bei Bedarf), rollenbasierte Passworte sind allen bekannt, Nichtnachvollziehbarkeit auf Nutzerebene, wer welche Änderungen (z.B. Konfiguration) zu welchem Zeitpunkt durchgeführt hat.

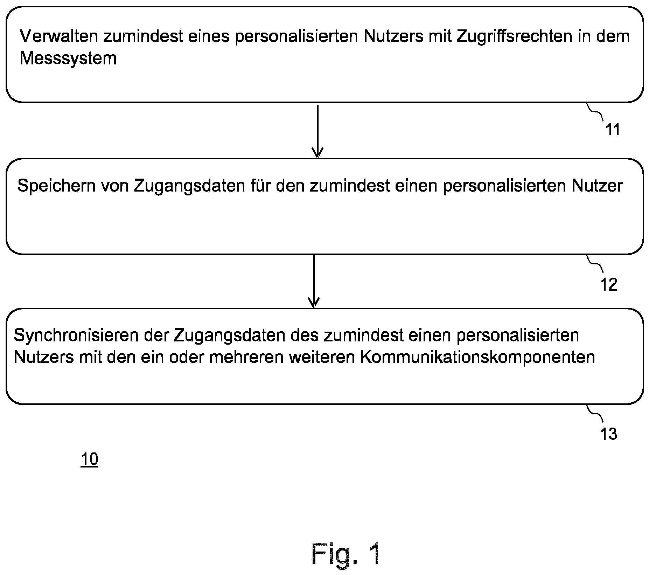

Fig. 1 zeigt ein Blockschaltbild eines Ausführungsbeispiels eines Verfahrens 10 für eine Kommunikationskomponente eines Messsystems. Das Messsystem weist ein oder mehrere weitere Kommunikationskomponenten auf. Das Verfahren 10 umfasst ein Verwalten 11 zumindest eines personalisierten Nutzers mit Zugriffsrechten in dem Messsystem. Verwalten in diesem Zusammenhang wird als ein Anlegen, Definieren, Löschen, Administrieren, Spezifizieren, Entziehen usw. von Nutzerdaten und Zugriffsrechten verstanden. Personalisierte Nutzer sind solche, die sich durch individuelle Zugangsdaten von vordefinierten Benutzergruppen oder Rollen, wie Standardnutzer, Gast und Administrator unterscheiden.

Wie Fig.1 weiter zeigt, umfasst das Verfahren 10 ein Speichern 12 von Zugangsdaten für den zumindest einen personalisierten Nutzer und dessen Zugriffsrechte. Dies kann beispielsweise auf einem Speichermedium jeglicher Art geschehen, Beispiele sind Festplatten, Netzwerkspeicher, Arbeitsspeicher, usw. Das Verfahren 10 umfasst ferner ein Synchronisieren 13 der Zugangsdaten des zumindest einen personalisierten Nutzers mit Zugriffsrechten mit den ein oder mehreren weiteren Kommunikationskomponenten. Unter dem Synchronisieren sei hier ein Abgleichen der Zugangsdaten derart verstanden, dass die Zugangsdaten nach dem Synchronisieren 13 an den beteiligten Komponenten gleich sind und beispielsweise der aktuellsten Fassung der Zugangsdaten entsprechen. Das Gleiche gilt für die Zugriffsrechte. Die Kommunikationskomponente umfasst einen Kontroller und die ein oder mehreren weiteren Kommunikationskomponenten umfassen ein oder mehrere Transmitter. Das Verfahren 10 umfasst ein Erhalten aktualisierter Zugangsdaten an den ein oder mehreren Transmittern oder an einer anderen mit dem Kontroller kommunizierenden Kommunikationskomponente, und ein Vereinheitlichen der aktualisierten Zugangsdaten unter den ein oder mehreren Kommunikationskomponenten.

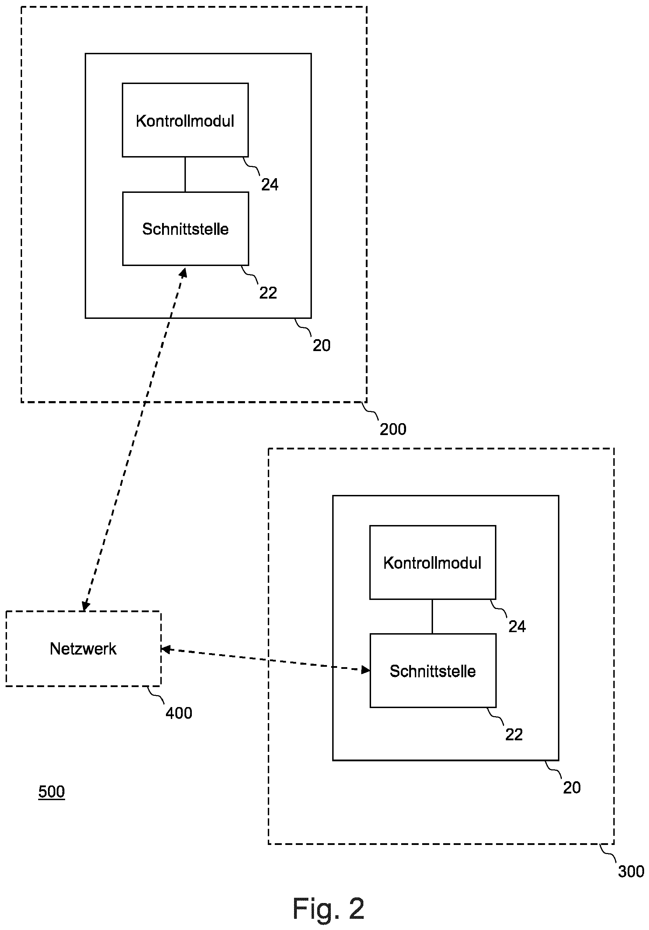

Fig. 2 zeigt ein Blockschaltbild eines Ausführungsbeispiels einer Vorrichtung 20 für eine Kommunikationskomponente 200, 300 eines Messsystems 500. Die Vorrichtung 20 für die Kommunikationskomponente 200, 300 des Messsystems 500 umfasst zumindest eine Schnittstelle 22, die zur Kommunikation in einem Netzwerk 400 ausgebildet ist. Die Vorrichtung 20 umfasst ferner ein Kontrollmodul 24, das mit der zumindest einen Schnittstelle 22 gekoppelt ist und das zur Durchführung eines der hierin beschriebenen Verfahren 10 ausgebildet ist. Fig. 2 illustriert darüber hinaus zwei Kommunikationskomponenten 200, 300, die jeweils ein Ausführungsbeispiel einer Vorrichtung 20 umfassen und die in gestrichelten Linien dargestellt sind, da diese aus Sicht der Vorrichtung 20 optional zu betrachten sind. Beispiele für solche Kommunikationskomponenten 200, 300 sind ein Kontroller, ein Transmitter oder ein Zugangsknoten für das Netzwerk 400, beispielsweise in einer lokalen Komponente oder auch in der Cloud. Das Netzwerk 400 kann beispielsweise ein IP (Internetprotokoll) Netzwerk oder ein auf einem digitalen Feldbus basierendes Netzwerk sein. Fig. 2 zeigt darüber hinaus ein Ausführungsbeispiel eines Messsystems 500, dass mehrere über das Netzwerk 400 kommunizierende Kommunikationskomponenten 200, 300 umfasst.

Die Schnittstelle 22 kann in Ausführungsbeispielen als typische Schnittstelle zur Kommunikation in Netzwerken 400 ausgebildet sein. Beispielsweise kann diese in Ausführungsbeispielen durch entsprechende Kontakte ausgebildet sein. Sie kann in Ausführungsbeispielen auch als separate Hardware ausgeführt sein und einen Speicher umfassen, der die zu sendenden beziehungsweise die empfangenen Signale zumindest vorübergehend speichert. Die Schnittstelle 22 kann zum Empfang von elektrischen Signalen ausgebildet sein, zum Beispiel als Busschnittstelle, als optische Schnittstelle, als Ethernet-Schnittstelle, als Funkschnittstelle, als Feldbusschnittstelle, HART-Schnittstelle, usw. Sie kann darüber hinaus in Ausführungsbeispielen zur Funkübertragung ausgebildet sein und ein Radio-Frontend sowie zugehörige Antennen umfassen. Weiter können für die ein oder mehreren Verbindungsarten Synchronisationsmechanismen zur Synchronisierung mit dem jeweiligen Übertragungsmedium umfassen.

Das Kontrollmodul 24 kann in Ausführungsbeispielen ein oder mehrere beliebige Kontroller, Mikrokontroller, Netzwerkprozessoren, Prozessorkerne, wie Digitale Signal Prozessorkerne (DSPs), programmierbare Hardwarekomponenten, usw. umfassen. Ausführungsbeispiele sind dabei nicht auf einen bestimmten Typ von Prozessorkern eingeschränkt. Es sind beliebige Prozessorkerne oder auch mehrere Prozessorkerne oder Mikrokontroller zur Implementierung eines Kontrollmoduls 24 denkbar. Es sind auch Implementierungen in integrierter Form mit anderen Vorrichtungen denkbar, beispielsweise in einer Steuereinheit die zusätzlich noch ein oder mehrere andere Funktionen umfasst. In Ausführungsbeispielen kann ein Kontrollmodul 24 durch einen Prozessorkern, einen Computerprozessorkern (CPU = Central Processing Unit), einen Grafikprozessorkern (GPU = Graphics Processing Unit), einen anwendungsspezifischen integrierten Schaltkreiskern (ASIC = Application-Specific Integrated Circuit), einen integrierten Schaltkreis (IC = Integrated Circuit), ein Ein-Chip-Systemkern (SOC = System on Chip), ein programmierbares Logikelement oder ein feldprogrammierbares Gatterarray mit einem Mikroprozessor (FPGA = Field Programmable Gate Array) als Kern des Bausteins oder der Bausteine realisiert sein.

Ausführungsbeispielen können allgemein in beliebigen Messsystemen zum Einsatz kommen.

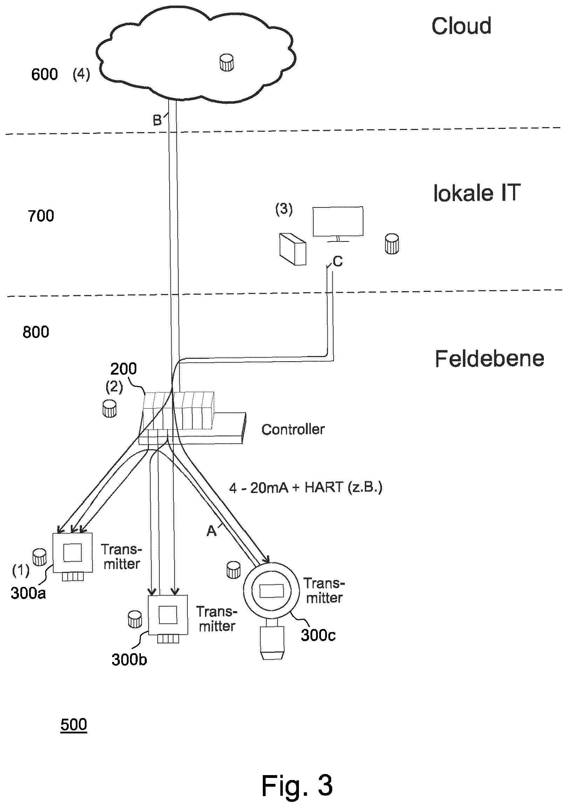

Im Folgenden wird insbesondere auf die Anwendung in Gaswarnanlagen eingegangen. Diese bestehen i. d. R. aus einem oder mehreren Sensoren oder Transmittern, in Verbindung mit einer oder mehreren Auswerteeinheiten (Kontroller) und ggf. der Anbindung an eine lokale IT (Informationstechnik) oder Cloud. Das Messsystem 500 umfasst demnach eine Gasmessanlage und eine Gaswarnanlage. Fig. 3 zeigt ein Blockschaltbild eines Ausführungsbeispiels eines Messsystems 500. Die Warnungsfunktion (schalten einer Gegenmaßnahme) ist in der Fig. 3 nicht konkret ausgeführt. Hierzu kann es vom Kontroller 200 aus, eine Verbindung zu Aktoren geben, die z.B. eine optische oder akustische Warnung oder einen Alarm erzeugen.

Das Messsystem 500 ist in diesem Ausführungsbeispiel in drei Ebenen unterteilt, ein Cloudebene 600 oben (im Sinne von Netzwerkcloud), eine lokale IT-Ebene 700 in der Mitte, und eine Feldebene 800 unten. In der Cloudebene 600 befindet sich eine Netzwerkcloud 600, die sich beispielsweise aus mehreren räumlich verteilt angeordneten Computern, Servern und/oder Zugangspunkten zusammensetzt. Hier können beispielsweise externe Zugangspunkte für ein Messsystem 500 implementiert werden.

Auf der lokalen IT-Ebene 700 können beispielsweise Zugänge durch lokale Rechner, d.h. mit dem Messsystem 500 am gleichen Standort befindliche Computer, vorhanden sein. Auf der Feldebene 800 befindet sich in diesem Ausführungsbeispiel zumindest ein Kontroller 200 und drei Transmitter/Sensoren 300a, 300b und 300c.

Die Fig. 3 illustriert dabei Kommunikationskomponenten 200, 300a, 300b, 300c, auf der Feldebene 800 sowie Kommunikationskomponenten, beispielsweise Rechner oder Zugangsknoten, auf den Ebenen 700 und 800 für das Messsystem 500 mit jeweils einer Vorrichtung 20. Die Kommunikationskomponente 200 entspricht dabei einem Kontroller und die Kommunikationskomponenten 300a, 300b und 300c entsprechen dabei Transmittern 300a, 300b und 300c. Demnach umfasst zumindest eine erste Kommunikationskomponente einen Kontroller 200 und zumindest eine zweite Kommunikationskomponente einen Transmitter 300abc.

Ein Sensor bzw. Transmitter 300a, 300b und 300c übernimmt die Messung der Gaskonzentration und leitet diese über z.B. 4-20mA in Verbindung mit einem digitalen Bus (z.B. HART, aber auch wireless) an den Kontroller 200 weiter. Bei der Verbindung sind in Ausführungsbeispielen viele Varianten denkbar. Beispiele sind 4-20mA allein (dann nicht digital) oder mit HART, andere Kommunikationsprotokolle wie Profibus aber auch Wireless-Protokolle (laufen i.d.R. ohne 4-20mA). Der Kontroller 200 übernimmt die Auswertung der Signale und schaltet Gegenmaßnahmen (z.B. akustische Alarmsingale) bei Erreichung einer definierten Schwelle. Der Kontroller 200 übernimmt auch die Weiterleitung der Daten z.B. in eine lokale IT 700 oder Cloudanwendung 600, wo weitere Auswertungen bzw. Konfigurationen vorgenommen werden können. In dem Messsystem 500 wird dann das Verfahren 10 zur Anwendung einer einheitlichen Benutzerverwaltung für das Messsystem 500 durchgeführt. Da kann das Synchronisieren 13 ferner ein Synchronisieren der Zugriffsdaten und Zugriffsrechte des zumindest einen personalisierten Nutzers umfassen. Die Zugriffrechte können auf einer dem zumindest einen personalisierten Nutzer zugewiesenen Rolle basieren, beispielsweise als modifizierter Gastnutzer.

Wie die Fig. 3 zeigt, umfasst das Messsystem 500 zumindest einen Kontroller 200 und ein oder mehrere mit dem Kontroller 200 digital kommunizierende Transmitter 300abc. Die Transmitter 300abc sind vorliegend mit Gasmesssensoren gekoppelt. Das Synchronisieren 13 erfolgt hier zumindest teilweise über einen digitalen Feldbus zwischen dem Kontroller 200 und den Transmittern 300abc (z.B. 4-20mA mit HART). Darüber hinaus können zumindest einige der beteiligten Komponenten auf allen drei Ebenen 600, 700, 800 eine Änderung von Zugangsdaten dokumentieren.

Beispielsweise erhält der Kontroller 200 von einem Rechner der lokalen IT 700 über ein IP-Netzwerk aktualisierte Nutzerdaten für einen bestimmten Nutzer. Das Synchronisieren 13 der Zugangsdaten wird dann durch Vereinheitlichen der Zugangsdaten an den mehreren Transmittern 300abc durch den Kontroller 200 erreicht. Die aktualisierten Zugangsdaten und ein Vereinheitlichen der aktualisierten Zugangsdaten wird somit an dem Kontroller 200 und den ein oder mehreren Transmittern 300abc durchgeführt. In weiteren Ausführungsbeispielen können die aktualisierten Zugangsdaten auch an beliebigen anderen Zugangspunkten des Messsystems 500 erhalten werden. Beispielsweise kann das Erhalten der aktualisierten Zugangsdaten an dem Kontroller 200, an einem Transmitter 300abc oder an einer anderen mit dem Kontroller 200 kommunizierenden Kommunikationskomponente erfolgen.

Die andere mit dem Kontroller 200 kommunizierende Kommunikationskomponente kann beispielsweise eine Leitwarte in einem zu dem Messsystem lokalen IT-System 700 oder einen Zugangsknoten in einer Cloud 600 umfassen. Messsysteme 500 verfügen zumeist über eine in unmittelbarer örtlicher Umgebung installierte Administration (Wartung, Verwaltung, Betreuung, usw.), wie z.B. eine lokale IT 700. Es ist aber auch denkbar, dass ein Messsystem ergänzend oder alternativ von einem entfernteren Knoten aus administriert, gewartet oder betreut werden. Dabei kann es sich um einen über entsprechende Sicherheitsmechanismen, wie z.B. Verschlüsselung, VPN (von engl. Virtual Private Network), Protokolltunnel, angebundenen Computer, die Zugriff auf das Messsystem 500 haben. Dabei kann es sich prinzipiell um einen beliebigen Zugangsknoten in der Cloud 600 handeln. Zumindest in manchen Ausführungsbeispielen werden dann die aktualisierten Zugangsdaten an allen für einen Nutzerzugang geeigneten Kommunikationskomponenten auf allen Ebenen 600, 700, 800 vereinheitlicht. Dies kann sich ebenso auf die Zugriffsrechte beziehen, sodass das Verfahren 10 auch ein Verwalten und/oder Vereinheitlichen von personalisierten Zugriffsrechten für den zumindest einen personalisierten Nutzer umfasst.

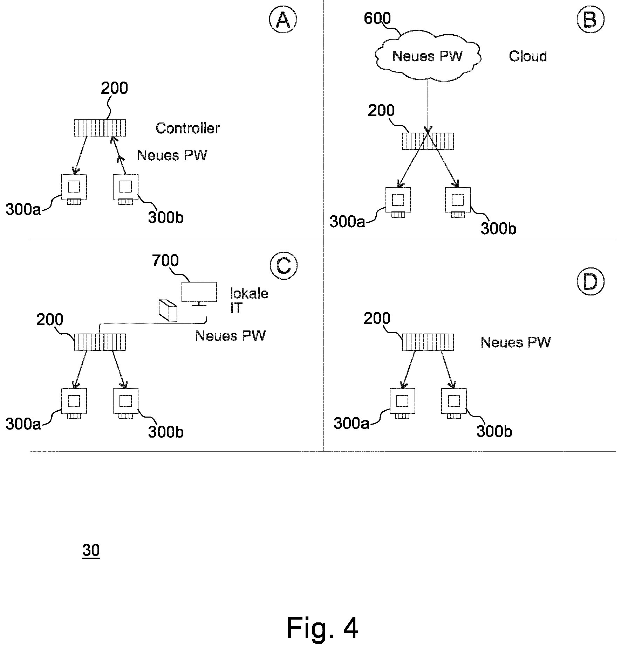

Fig. 4 zeigt mögliche Kommunikationsszenarien in einem Ausführungsbeispiel. In einem ersten Szenario A, das in der Fig. 4 links oben dargestellt ist, empfängt der Transmitter 300b ein neues Passwort, welches an den Kontroller 200 kommuniziert wird. Der Kontroller gibt das neue Passwort dann an den Transmitter 300a weiter und erreicht so eine Synchronisation. Dabei kann beispielsweise ein Feldbus wie HART verwendet werden. Gleichzeitig kann das Passwort auch an weitere Kommunikationskomponenten auf den Ebenen darüber weitergegeben werden.

Ein weiteres Szenario B ist in der Fig. 4 rechts oben dargestellt. Hier wird an einem Zugangsknoten oder einer Kommunikationskomponente in der Cloud 600 ein neues Passwort vergeben, dass dann über die Cloud an den Kontroller 200 kommuniziert wird. Der Kontroller 200 gibt das neue Passwort dann an die Transmitter 300a und 300b weiter und synchronisiert diese damit auf.

Die Fig. 4 zeigt links unten ein Szenario C bei dem ein Rechner der lokalen IT 700 ein neues Passwort erhält und diese an den Kontroller 200 kommuniziert wird. Der Kontroller 200 informiert dann wiederum die beiden Transmitter 300a und 300b, um die Synchronisation der Zugangsdaten zu erreichen.

In einem Szenario D, das in der Fig. 4 rechts unten dargestellt ist, wird das neue Passwort direkt an dem Kontroller 200 eingegeben und dieser synchronisiert die beiden Transmitter 300a und 300b sowie ggf. weitere Kommunikationskomponenten auf den Ebenen 600 und 700.

Eine globale Nutzerverwaltung für Messsysteme 500 ermöglicht es, von jedem Zugriffspunkt aus (Transmitter 300abc, Kontroller 200, Leitwarte über lokale IT 700, Cloud 800) über dieselben, für den Nutzer personalisierten, Nutzerdaten auf die entsprechende Komponente zuzugreifen. Dazu können an jedem Zugriffspunkt die Nutzerdaten (Nutzer, Rolle, Passwort, etc.) verschlüsselt abgelegt werden. Eine Synchronisierung wird bei Änderungen, Neustarts oder zeitgesteuert vorgenommen. Dies kann in weiteren Ausführungsbeispielen so gestaltet sein, dass sich die Nutzerverwaltung auf definierte Gruppen von Einzelkomponenten bezieht, beispielsweise bei allen Sensoren und Transmittern die messtechnisch den gleichen Messzweck erfüllen aber an verschiedenen Orten installiert sind.

Die folgenden Szenarien beschreiben mögliche Anwendungen, vgl. Fig. 4:
(A, links oben) 2 Transmitter 300a, 300b mit Kontroller 200, keine Cloud: Im Transmitter 300b wird ein neues Passwort für einen Nutzer eingestellt, dieses wird auch mit dem Kontroller 200 synchronisiert, und anschließend mit allen weiteren Transmittern 300a;
(B, rechts oben) 2 Transmitter 300a, 300b mit Kontroller 200 und Cloud 600: In der Cloud 600 wird ein neuer Nutzer mit Passwort, Rolle etc. angelegt. Anschließend erfolgt eine Synchronisation mit Kontroller 200 und Transmittern 300a, 300b;
(C, links unten) 2 Transmitter 300a, 300b mit Kontroller 200 und Applikation in lokaler IT 700: In der Applikation wird ein neuer Nutzer mit Passwort, Rolle etc. angelegt. Anschließend erfolgt eine Synchronisation mit Kontroller 200 und Transmittern 300a, 300b;
(D, rechts unten) 2 Transmitter 300a, 300b mit Kontroller 200, keine Cloud: Im Kontroller 200 wird ein neues Passwort für einen Nutzer eingestellt, anschließend erfolgt eine Synchronisation mit angeschlossenen Transmittern 300a, 300b.
(E) (kein Bild): Zeitliche und räumliche Berechtigungen für bestimmte Applikationen möglich. Dabei kann eine zeitliche Berechtigung eine zeitlich limitierte Zugangsberechtigung für einen Nutzer umfassen, z.B. für eine Minute, 5 Minuten, 10 Minuten, eine Stunde, einen Tag, usw. Die zeitlich limitierte Berechtigung kann sich auch auf genau einen Zugang beispielsweise für eine Minute, 5 Minuten, 10 Minuten, eine Stunde, usw. beziehen. Eine räumliche Berechtigung kann sich auf eine Untergruppe der Kommunikationskomponenten eines Systems beziehen, beispielsweise alle Transmitter/Kontroller in einem Teilbereich des Systems, wie beispielsweise entlang einer bestimmten Leitung oder in einem bestimmten Prozessbereich.

Neben zeitlich oder räumlich limitierten Berechtigungen können in weiteren Ausführungsbeispielen auch funktional limitierte Berechtigungen oder Berechtigungsvergaben vorkommen. Beispielsweise kann in Ausführungsbeispielen vorgesehen sein, dass Berechtigungen nur für bestimmte Typen von Transmittern/Sensoren oder Kommunikationskomponenten vergeben werden. Diese funktionale Begrenzung kann bei Wartungsarbeiten hilfreich sein, beispielsweise wenn nur bestimmte Typen gewartet werden. In einem Ausführungsbeispiel können so beispielsweise in einer Gasmessanlage verschiedene Gassensoren implementiert sein, z.B. Sauerstoffsensoren und Chlorsensoren. Sollen nur die Sauerstoffsensoren gewartet werden, so kann es vorkommen, dass speziell nur für die Sauerstoffsensoren gewisse, zur Wartung notwendige, Zugriffsrechte vergeben werden sollen. Insofern kann eine Vereinheitlichung der aktualisierten Zugangsdaten auf die Sauerstoffsensoren begrenzt werden.

Die ein oder mehreren Kommunikationskomponenten können insofern auch Transmitter oder Sensoren mit einer bestimmten Funktionalität oder eines bestimmten Typs umfassen. In manchen Ausführungsbeispielen kann dann das Vereinheitlichen der aktualisierten Zugangsdaten unter den ein oder mehreren Kommunikationskomponenten, ein Vereinheitlichen der Zugangsdaten unter Kommunikationskomponenten in einem bestimmten räumlichen Bereich, in einem Verwaltungsbereich (z.B. Adressraum), für einen bestimmten Zeitraum und/oder mit einer bestimmten Funktionalität umfassen.

In den bisherigen Ausführungsbeispielen erfolgt eine Synchronisation der Transmitter 300abc durch den Kontroller 200. In anderen Ausführungsbeispielen können auch andere Komponenten oder ein Transmitter selbst die Aktualisierung weiterer Transmitter koordinieren bzw. auslösen. Dies kann beispielsweise durch Rundfunknachrichten (Broadcast-Nachrichten) an alle Komponenten in einem System geschehen, die prinzipiell von jedem autorisierten Zugangsknoten oder Kommunikationskomponente aus versendet werden können.

Ausführungsbeispiele können weiterhin ein Computerprogramm mit einem Programmcode zum Ausführen eines oder mehrerer der obigen Verfahren sein oder sich darauf beziehen, wenn das Computerprogramm auf einem Computer oder Prozessor ausgeführt wird. Schritte, Operationen oder Prozesse von verschiedenen, oben beschriebenen Verfahren können durch programmierte Computer oder Prozessoren ausgeführt werden. Beispiele können auch Programmspeichervorrichtungen, z. B. Digitaldatenspeichermedien, abdecken, die maschinen-, prozessor- oder computerlesbar sind und maschinenausführbare, prozessorausführbare oder computerausführbare Programme von Anweisungen codieren. Die Anweisungen führen einige oder alle der Schritte der oben beschriebenen Verfahren aus oder verursachen deren Ausführung. Die Programmspeichervorrichtungen können z. B. Digitalspeicher (Flash-Speicher oder Solid State Drive-Speicher), magnetische Speichermedien wie beispielsweise Magnetplatten und Magnetbänder, Festplattenlaufwerke oder optisch lesbare Digitaldatenspeichermedien umfassen oder sein. Weitere Beispiele können auch Computer, Prozessoren oder Steuereinheiten, die zum Ausführen der Schritte der oben beschriebenen Verfahren programmiert sind, oder (feld-) programmierbare Logik-Arrays ((F)PLAs = (Field) Programmable Logic Arrays) oder (feld-)programmierbare Gate-Arrays ((F)PGA = (Field) Programmable Gate Arrays), die zum Ausführen der Schritte der oben beschriebenen Verfahren programmiert sind, abdecken.

Funktionen verschiedener in den Figuren gezeigter Elemente sowie die bezeichneten Funktionsblöcke können in Form dedizierter Hardware, z. B "eines Signalanbieters", "einer Signalverarbeitungseinheit", "eines Prozessors", "einer Steuerung" etc. sowie als Hardware fähig zum Ausführen von Software in Verbindung mit zugehöriger Software implementiert sein. Bei Bereitstellung durch einen Prozessor können die Funktionen durch einen einzelnen dedizierten Prozessor, durch einen einzelnen gemeinschaftlich verwendeten Prozessor oder durch eine Mehrzahl von individuellen Prozessoren bereitgestellt sein, von denen einige oder von denen alle gemeinschaftlich verwendet werden können. Allerdings ist der Begriff "Prozessor" oder "Steuerung" bei Weitem nicht auf ausschließlich zur Ausführung von Software fähige Hardware begrenzt, sondern kann Digitalsignalprozessor-Hardware (DSP-Hardware; DSP = Digital Signal Processor), Netzprozessor, anwendungs-spezifische integrierte Schaltung (ASIC = Application Specific Integrated Circuit), feldprogrammierbare Logikanordnung (FPGA = Field Programmable Gate Array), Nurlesespeicher (ROM = Read Only Memory) zum Speichern von Software, Direktzugriffsspeicher (RAM = Random Access Memory) und nichtflüchtige Speichervorrichtung (storage) umfassen. Sonstige Hardware, herkömmliche und/oder kundenspezifische, kann auch eingeschlossen sein.

Ein Blockdiagramm kann zum Beispiel ein grobes Schaltdiagramm darstellen, das die Grundsätze der Offenbarung implementiert. Auf ähnliche Weise können ein Flussdiagramm, ein Ablaufdiagramm, ein Zustandsübergangsdiagramm, ein Pseudocode und dergleichen verschiedene Prozesse, Operationen oder Schritte repräsentieren, die zum Beispiel im Wesentlichen in computerlesbarem Medium dargestellt und so durch einen Computer oder Prozessor ausgeführt werden, ungeachtet dessen, ob ein solcher Computer oder Prozessor explizit gezeigt ist. In der Beschreibung oder in den Patentansprüchen offenbarte Verfahren können durch ein Bauelement implementiert werden, das ein Mittel zum Ausführen eines jeden der jeweiligen Schritte dieser Verfahren aufweist.

Es versteht sich, dass die Offenbarung mehrerer, in der Beschreibung oder den Ansprüchen offenbarter Schritte, Prozesse, Operationen oder Funktionen nicht als in der bestimmten Reihenfolge befindlich ausgelegt werden soll, sofern dies nicht explizit oder implizit anderweitig, z. B. aus technischen Gründen, angegeben ist. Daher werden diese durch die Offenbarung von mehreren Schritten oder Funktionen nicht auf eine bestimmte Reihenfolge begrenzt, es sei denn, dass diese Schritte oder Funktionen aus technischen Gründen nicht austauschbar sind. Ferner kann bei einigen Beispielen ein einzelner Schritt, Funktion, Prozess oder Operation mehrere Teilschritte, -funktionen, -prozesse oder -operationen einschließen und/oder in dieselben aufgebrochen werden. Solche Teilschritte können eingeschlossen sein und Teil der Offenbarung dieses Einzelschritts sein, sofern sie nicht explizit ausgeschlossen sind.

### Bezugszeichenliste

- 10: Verfahren für eine Kommunikationskomponente eines Messsystems
- 11: Verwalten zumindest eines personalisierten Nutzers mit Zugriffsrechten in dem Messsystem
- 12: Speichern von Zugangsdaten für den zumindest einen personalisierten Nutzer
- 13: Synchronisieren der Zugangsdaten des zumindest einen personalisierten Nutzers mit den ein oder mehreren weiteren Kommunikationskomponenten

- 20: Vorrichtung für eine Kommunikationskomponente
- 22: Schnittstelle
- 24: Kontrollmodul

- 200: Kontroller, Kommunikationskomponente

- 300: Kommunikationskomponente
- 300a: Transmitter, Kommunikationskomponente
- 300b: Transmitter, Kommunikationskomponente
- 300c: Transmitter, Kommunikationskomponente

- 400: Netzwerk

- 500: Messsystem

- 600: Lokale IT, Kommunikationskomponente

- 700: Cloud, Kommunikationskomponente

- 800: Feldebene, Kommunikationskomponente

## Patentansprüche

1. Verfahren (10) für eine Kommunikationskomponente (200; 300a; 300b; 300c; 600; 700) eines Messsystems (500), wobei das Messsystem (500) ein oder mehrere weitere Kommunikationskomponenten (200; 300a; 300b; 300c; 600; 700) aufweist, mit
Verwalten (11) zumindest eines personalisierten Nutzers mit Zugriffsrechten in dem Messsystem (500);
Speichern (12) von Zugangsdaten für den zumindest einen personalisierten Nutzer; und
Synchronisieren (13) der Zugangsdaten des zumindest einen personalisierten Nutzers mit den ein oder mehreren weiteren Kommunikationskomponenten (200; 300a; 300b; 300c; 600; 700)
wobei die Kommunikationskomponente (200; 300a; 300b; 300c; 600; 700) einen Kontroller (200) und die ein oder mehreren weiteren Kommunikationskomponenten (200; 300a; 300b; 300c; 600; 700) ein oder mehrere Transmitter (300a; 300b; 300c) umfassen,
ferner umfassend
Erhalten aktualisierter Zugangsdaten an den ein oder mehreren Transmittern (300a; 300b; 300c) oder an einer anderen mit dem Kontroller (200) kommunizierenden Kommunikationskomponente, und
Vereinheitlichen der aktualisierten Zugangsdaten unter den ein oder mehreren Kommunikationskomponenten (200; 300a; 300b; 300c; 600; 700).

2. Verfahren (10) gemäß Anspruch 1, wobei das Messsystem (500) eine Gasmessanlage umfasst.

3. Verfahren (10) gemäß einem der Ansprüche 1 bis 2, wobei das Synchronisieren (13) ferner ein Synchronisieren der Zugriffsrechte des zumindest einen personalisierten Nutzers umfasst.

4. Verfahren (10) gemäß einem der Ansprüche 1 bis 3, wobei die Zugriffrechte auf einer dem zumindest einen personalisierten Nutzer zugewiesenen Rolle basieren.

5. Verfahren (10) gemäß einem der Ansprüche 1 bis 4, wobei das Messsystem (500) zumindest einen Kontroller (200) und ein oder mehrere mit dem Kontroller (200) digital kommunizierende Transmitter (300a; 300b, 300c) umfasst, wobei die Transmitter (300a; 300b; 300c) mit Gasmesssensoren gekoppelt sind.

6. Verfahren (10) gemäß einem der Ansprüche 1 bis 5, wobei das Synchronisieren (13) zumindest teilweise über einen digitalen Feldbus erfolgt.

7. Verfahren (10) gemäß einem der Ansprüche 1 bis 6, ferner umfassend Dokumentieren von Änderungen der Zugangsdaten.

8. Verfahren gemäß einem der Ansprüche 1 bis 7, wobei die Zugriffsrechte des Nutzers zeitlich und/oder räumlich limitiert sind.

9. Verfahren (10) gemäß einem der Ansprüche 1 bis 8, ferner umfassend Verwalten von personalisierten Zugriffsrechten für den zumindest einen personalisierten Nutzer.

10. Computerprogramm mit einem Programmcode zur Durchführung eines der Verfahren (10) gemäß einem der Ansprüche 1 bis 9, wenn der Programmcode auf einem Computer, einem Prozessor oder einer programmierbaren Hardwarekomponente ausgeführt wird.

11. Vorrichtung (20) für eine Kommunikationskomponente (200; 300a; 300b; 300c; 600; 700) eines Messsystems (500), mit
einer Schnittstelle (22), die zur Kommunikation in einem Netzwerk (400) ausgebildet ist; und
einem Kontrollmodul (24), das zur Durchführung eines der Verfahren (10) gemäß einem der Ansprüche 1 bis 9 ausgebildet ist.

12. Kommunikationskomponente (200; 300a; 300b; 300c; 600; 700) für ein Messsystem (500) mit einer Vorrichtung (20) gemäß Anspruch 11.

13. Messsystem (500) mit mehreren Kommunikationskomponenten (200; 300a; 300b; 300c; 600; 700) gemäß Anspruch 12, wobei zumindest eine erste Kommunikationskomponente (200; 300a; 300b; 300c; 600; 700) einen Kontroller (200) umfasst und zumindest eine zweite Kommunikationskomponente (200; 300a; 300b; 300c; 600; 700) einen Transmitter (300a; 300b; 300c) umfasst.

14. Messsystem (500) gemäß Anspruch 13, das eine Gasmessanlage umfasst.
